# EUROPEAN PATENT APPLICATION

(11) **EP 2 481 347 A1**
(43) Date of publication of application: **01.08.2012**
(21) Application number: 12151149.7
(22) Date of filing: 13.01.2012
(51) Int. Cl.: A61B 5/00, A61F 7/12

(54) **Device for sensitivity testing**

(30) Priority: 27.01.2011 GB 201101390; 08.07.2011 GB 201111780
(71) Applicant: Barts And The London NHS Trust, Whitechapel London Greater London E1 1BB (GB)
(72) Inventor: Reeves, Jonathan William, Cheshunt, Hertfordshire EN8 9BX (GB); Birch, Malcolm John, Kings Langley, Hertfordshire WD4 9QA (GB)
(74) Representative: Forsythe, Dominic

(57) **Abstract**

Devices and methods for sensitivity testing are provided. Disclosed devices include a flexible catheter body having a distal end and a proximal end, a peltier element, a contact element located at or near the distal end of the catheter body that can be heated or cooled by applying a voltage to the peltier element, and a contact sensor for detecting contact between a contact surface of said contact element and tissue to be tested.

## Description

The present invention relates to devices and methods for measuring tissue sensitivity.

Measurement of tissue sensitivity is important in the assessment and differentiation of clinical conditions such as Gastrointestinal Oesophageal Reflux Disease (GORD). GORD occurs where acid from the stomach comes back up into the oesophagus leading to burning sensations, chest pain and difficulty breathing. Long term acid erosion of the oesophagus can lead to serious complications and morbidity, so that early intervention is necessary. Treatment for GORD usually consists of proton pump inhibitors (PPIs) as they provide powerful gastric acid control. However, up to 40% of patients diagnosed with GORD and subsequently treated fail to respond symptomatically to standard doses of treatment.

Oesophageal hypersensitivity (OH) is a condition where GORD-like symptoms arise but the levels of acid in the gullet are normal or mild. Patients with oesophageal hypersensitivity have an increased perception of normal stimuli. Treatment with PPIs will have little effect on this patient subset, so there is a requirement for a definitive test to rule out OH when testing for GORD.

Devices that use electric shocks to stimulate tissue, or balloons which can be inflated to mimic distension sensation are available, but have not so far proved effective for carrying out accurate sensitivity measurements of the type required. Heat sensitivity is an alternative approach which has shown promise for diagnosing visceral hypersensitivity (OLESEN SS, et al - 2009 - An endoscopic method for thermal and chemical stimulation of the human oesophagus - Neurogastroenterol Motil 1250 - e116). Current experimental heating devices use water filled balloon systems to provide the heat source. This method has several disadvantages. For example, the water is heated outside the body and then pumped into the balloon, which could cause unwanted heating of tissue surrounding the target site and skew results. Also, as the water is pumped into the balloon from the outside, it will lose temperature, hence measurements may not be accurate, again skewing results.

WO9963888 discloses a device using a peltier thermoelectric heat pump which allows accurate and rapid heating of target tissues. The device is rigid enough to allow the user to press the device against tissue to ensure good contact with the heating element. Thus the device can only be used to assess tissues where there is a direct line of access from the operator, i.e. rectally, vaginally or orally, to limited depths. As the oesophagus is not straight, the rigid nature of this device would ensure that it could not be used in the oesophagus.

These prior art devices also require a response from the patient to gauge perception of temperature and thus sensitivity. This approach is thus highly subjective and open to variation from patient to patient.

It is an object of the invention to at least partially overcome one or more of the problems of the prior art discussed above.

According to an aspect of the invention, there is provided a device for sensitivity testing, comprising: a flexible catheter body having a distal end and a proximal end; a peltier element; a contact element located at or near the distal end of the catheter body that can be heated or cooled by applying a voltage to the peltier element; and a contact sensor for detecting contact between a contact surface of said contact element and tissue to be tested.

The flexible catheter body allows the peltier-driven contact element and associated contact surface to be positioned at a site of interest while being well tolerated by the patient. The provision of the contact sensors makes it possible to evaluate the extent to which adequate thermal contact is made between the contact surface and tissue to be tested. It is thus possible to apply heating or cooling to tissue selectively in a highly controlled and reliable manner even in regions that are difficult to access, such as the oesophagus.

The device may further be provided with a pH sensor for measuring the level of acidity at or near the region where the contact surface is applying heating or cooling. Information from the pH sensor can be taken into account to assess the extent to which any response from the patient is a result of a combination of the effect of heating or cooling via the contact surface and the effects of excess acidity, for example.

A temperature sensor or plurality of temperature sensors may be provided for measuring the temperature of the contact element. Temperature measurements of the contact element can be used on their own, or in combination with data representing the power and/or current being applied to the peltier element as a reliable indicator of the amount of stimulus being applied to the patient.

The device may comprise a plurality of peltier elements within a single heating/cooling assembly and/or a plurality of heating/cooling assemblies each comprising one or more peltier elements.

The heating/cooling assemblies may be configured to provide heating or cooling through a wide range of azimuthal angles. Preferably, the contact surface or surfaces form(s) an azimuthally continuous ring and is/are heated or cooled through 360°. This arrangement helps to ensure that the heating or cooling is transferred reliably from the device to the tissue to be tested with a minimum of manual manipulation and/or repositioning of the device being necessary to establish a satisfactory thermal connection (as measured by the contact sensors).

The dimensions and/or mechanical properties of the device are preferably chosen so that the device can be inserted into the oesophagus orally while being well tolerated by the patient. Preferably, the dimensions are chosen such that the device can be inserted into the oesophagus nasally while being well supported by the patient.

According to an alternative aspect of the invention, there is provided a method of performing sensitivity testing, comprising: inserting a flexible catheter body orally or nasally into a patient so that a contact element located at or near a distal end of the catheter body and in thermal contact with a peltier element is brought to a region to be tested within the oesophagus, the peltier element being capable of heating or cooling the contact element; sensing whether a contact surface of the contact element is in contact with tissue at the region to be tested; applying a voltage to the peltier element in order to apply heating or cooling to the tissue at the region to be tested if it is detected in said sensing step that the contact surface of the contact element is in contact with tissue at the region to be tested.

Embodiments of the invention will now be described, by way of example only, with reference to the accompanying drawings in which corresponding reference symbols indicate corresponding parts, and in which:
Figure 1a is a schematic illustration of a portion of the human anatomy showing a region susceptible to GORD and an oral access route to the region;
Figure 1b depicts a device according to a disclosed embodiment that has been inserted orally so as to position a contact element at or near the region susceptible to GORD;
Figure 2a is a schematic illustration of a device according to a disclosed embodiment, showing a catheter body with pH sensor, electrical sensors and a heating/cooling assembly for heating or cooling tissue;
Figure 2b is a schematic illustration of a remote control handset for a patient, comprising a button (user operable means) for interrupting heating or cooling and a button (user operable means) for indicating a degree of sensation associated with heating or cooling;
Figure 2c is a schematic illustration of a remote control handset for an operator of the device other than the patient, for example a clinician or doctor, comprising a button (user operable means) for interrupting heating or cooling;
Figure 3a is a schematic side sectional view of a heating/cooling assembly;
Figure 3b is a schematic end sectional view of the heating/cooling assembly illustrated in Figure 3a;
Figure 4a is a schematic side sectional view of an alternative heating/cooling assembly, in which a plurality of peltier elements and contact elements provide heating or cooling in a 360° loop; and
Figure 4b is a schematic end sectional view of the heating/cooling assembly illustrated in Figure 4a;
Figure 5 is a schematic side sectional view of a device comprising a plurality of heating/cooling assemblies and a heat exchanger.

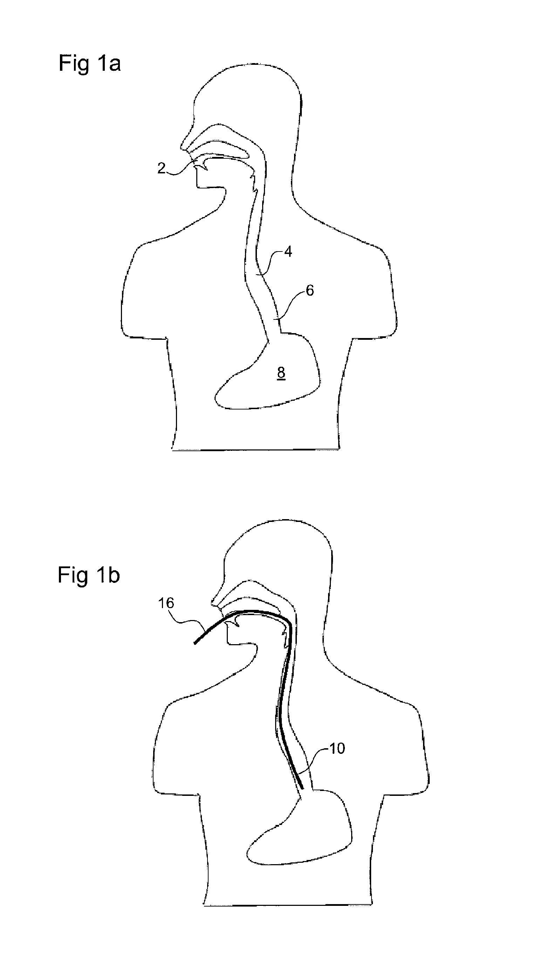

Figure 1a is a schematic illustration of the human anatomy showing the mouth 2, oesophagus 4, and stomach 8. Region 6 is a region that is particularly susceptible to Gastrointestinal Oesophageal Reflux Disease (GORD).

As explained earlier, it is important when attempting to diagnose GORD that oesophageal hypersensitivity (OH) is ruled out. The present disclosure presents devices which enable localized heating or cooling to be applied in a highly controlled and reliable manner in a variety of different internal regions, including those that are susceptible to GORD. The degree of sensitivity to such heating/cooling shown by the patient can be used to help decide whether OH is present and in what degree.

Figure 1b shows an example device 10 inserted orally into a position within a patient that allows assessment of OH at a region susceptible to GORD. The device 10 in this example is positioned within the patient by an insertion catheter 16, which is optionally detachable from the device 10.

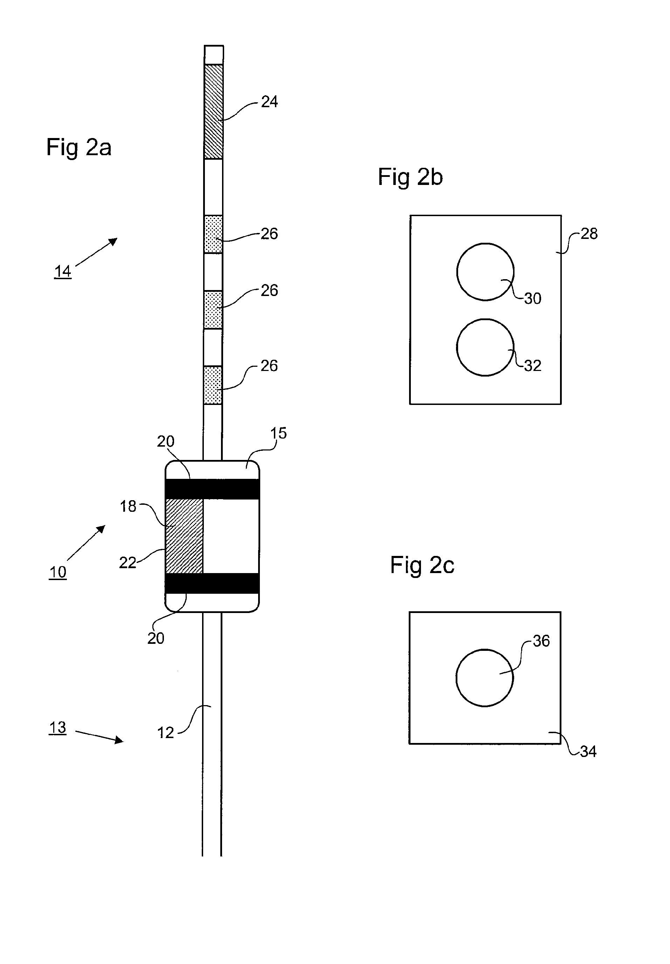

Figure 2a is a schematic side view showing the device 10 in further detail. The device 10 comprises a flexible catheter body 12 having a distal end 14 and a proximal end 13. The flexible catheter body 12 may for example have a tubular form. The flexible catheter body 12 may comprise an internal lumen providing access for electrical wiring. In this example arrangement, the flexible catheter body 12 supports a heating/cooling assembly 15. The heating/cooling assembly 15 comprises a contact element 18, which can be heated or cooled by a peltier element (described in further detail below). The contact element 18 is configured to present a contact surface 22 to the exterior of the device 10. When the device 10 is inserted into position within the patient (Figure 1b), the contact surface 22 is preferably arranged to be in contact with tissue at a site of interest. One or more contact sensors 20 may be provided for detecting whether the contact surface 22 is in contact with tissue. Preferably, the contact sensors 20 are also able to detect the pressure with which the contact surface 22 is pressed against tissue. Further preferably, the contact sensors 20 are able to detect what proportion of the contact surface 22 is in contact with tissue in the case where not all of the contact surface 22 is in contact with tissue.

The output from the contact sensors 20 can thus be used to assess the quality of the connection (i.e. the thermal conductivity associated with the connection) between the contact surface 22 and tissue. The output from the contact sensors 20 can thus be used to estimate the rate of transfer of heat to or from the tissue (depending on whether the peltier element is configured to heat or cool the tissue). This information can be used to improve the accuracy with which any patient response is interpreted.

The device 10 further comprises a plurality of electrical sensors 26 for detecting a patient's response to heating or cooling supplied by the heating/cooling assembly 15. The electrical sensors 26 may be configured to measure the local electrical impedance or resistivity of tissue for example. Alternatively or additionally, the electrical sensors 26 may be configured to measure one or more other electrical properties, such as inductance or capacitance. In the example shown, the electrical sensors 26 are evenly spaced longitudinally but this is not essential. In the example shown a plurality of electrical sensors 26 are provided, but in alternative configurations a single electrical sensor only may be used. In other configurations, there may be no electrical sensors (the clinician relying instead on the patient to give feedback consciously).

The example device 10 shown further comprises a pH sensor 24 for measuring the pH. The inclusion of a pH sensor 24 makes it possible to take into account the local pH in the region of interest when evaluating a patient's response to stimuli from the heating/cooling assembly. For example, a fall in pH to a level that might cause a burning sensation would be detected, and not falsely attributed to heating or cooling from the heating/cooling assembly 15.

In addition to, or instead of, the indication provided by the electrical sensors 26, a patient may be asked to provide feedback about the heating/cooling himself. One way in which this can be achieved efficiently is to provide the patient with a remote control handset 28 (part of a "remote control system"), as illustrated schematically in Figure 2b. The remote control handset 28 may comprise user operable means 30 (for example a button) allowing a user to provide an indication of the degree to which he is able to feel the heating/cooling provided by the device 10. For example, the device 10 may be configured to ramp up the rate of heating/cooling gradually (e.g. continuously or in steps) and the user may be asked to activate the user operable means when he is first able to detect the heating/cooling or when the heating/cooling first becomes uncomfortable. Alternatively or additionally, the system may be configured to vary the temperature of the contact surface 22 and/or the time for which a given elevated or depressed temperature is maintained randomly or pseudo randomly, for example in a series of pulses, with the patient being asked to provide an indication (e.g. press the user operable means 30 or use his voice) whenever he feels the stimulus (pulse).

The remote control handset 28 for the patient may also comprise an emergency cutout switch 32 which the patient can press in the event that he would like the heating/cooling to stop immediately.

A separate remote control handset 34 (Figure 2c) may be provided for the clinician. The clinician's remote control handset 34 may also comprise an emergency cutout switch 36.

The patient's remote control system 28 or the clinician's remote control system 34, or both, may be configured to communicate with a control system and/or power supply for the device 10, via any communication method, such as wirelessly or via wires.

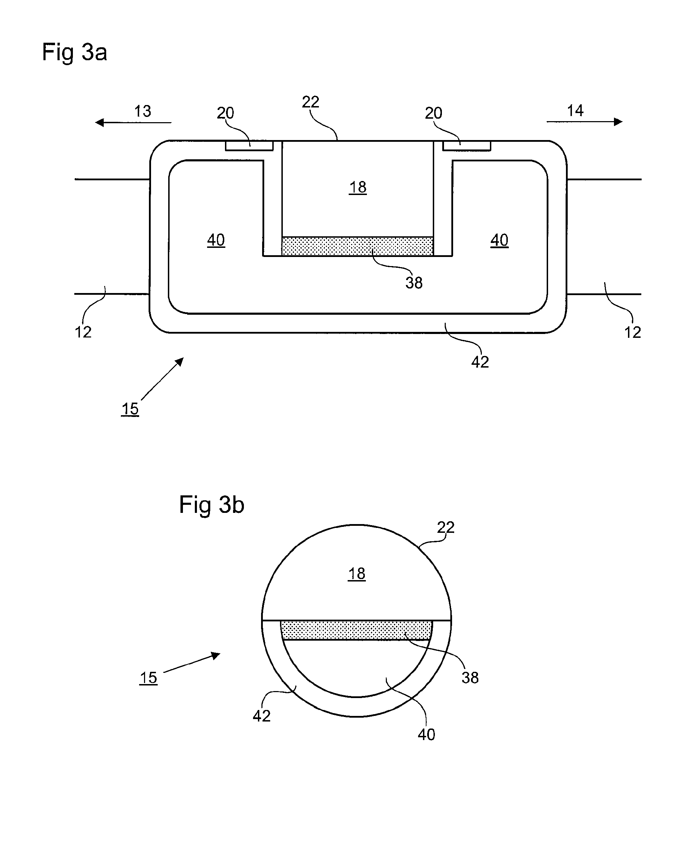

Figures 3a and 3b are side and end sectional views, respectively, showing in further detail an example configuration for the heating/cooling assembly 15. The heating/cooling assembly 15 comprises a miniature peltier thermoelectric heat pump, referred to here as a "peltier element" 38. The peltier element 38 is in contact with a contact element 18 on a first side and a heat sink 40 on the other side. When a voltage is applied to the peltier element 38, heat is "pumped" from the contact element 18 to the heat sink 40 or from the heat sink 40 to the contact element 18, depending on the sign of the applied voltage. The heat sink 40 is formed from a thermally conductive material such as copper, preferably also with a relatively high heat capacity. The heat sink 40 is prevented from coming into contact with tissue by a thermally insulating member 42 which substantially or completely surrounds the heat sink (or at least those portions of the heat sink that are not in contact with the peltier element 38 or other elements which act as a barrier between the heat sink 40 and surrounding tissue).

By controlling the power and/or current applied to the peltier element 38 it is possible to control the rate at which heat is transferred to or taken from the contact element 18. It is thus possible accurately to control the temperature of the contact surface 22 which is to be brought into contact with the tissue.

The thermally insulating member 42 may be formed from a plastic, for example PEEK (poly ether ether ketone).

In the example shown, the contact surface 22 is flush with an outer surface of the thermally insulating member 42. However, in alternative arrangements the contact surface 22 may be arranged to protrude, for example such that there is a step between the contact surface 22 and the surrounding regions of the outer surface of the thermally insulating member 42. Arranging for the contact surface 22 to protrude in this manner may help to encourage good thermal contact between the contact surface 22 and the tissue.

In the example shown, two contact sensors 20 are provided, one on either side of the contact surface 22. In alternative arrangements, only a single contact sensor 20 may be provided, for example on one side of the contact surface 22 only. In alternative configurations, more than two contact sensors 20 may be provided. Contact sensors may be configured to substantially surround the contact surface 22 in all directions. This could be achieved by providing a single contact sensor having a continuous sensing surface in the form of a loop, or by providing a plurality of contact sensors at regularly spaced intervals around the loop. Where a plurality of contact sensors 20 are provided, their readings may be analysed together in order to determine the quality of contact between the contact surface 22 and tissue, as discussed above.

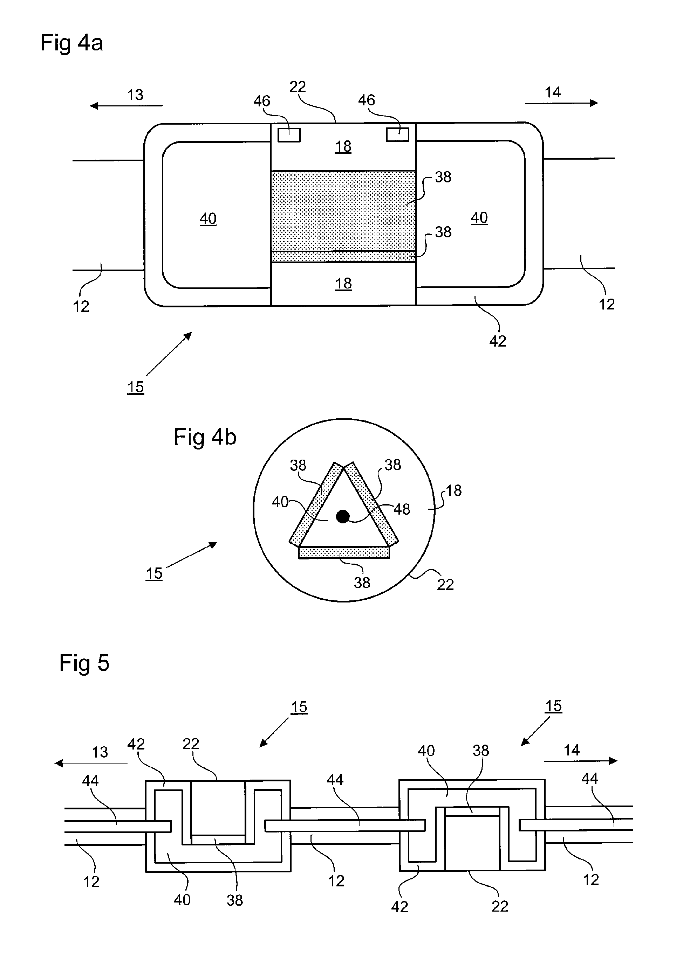

As can be seen from the end sectional view of Figure 3b, the contact surface 22 provides heating through 180° by means of a semicircular outer profile. This wide range of angles is advantageous because it increases the probability of a portion of the contact surface 22 being in good thermal contact with tissue for a given position of the device 10 within the patient. However, in certain situations it may be desirable to reduce the range of angles through which heating or cooling is provided, in order to provide more targeted heating. Alternatively, it may be desirable to increase the range of angles through which heating/cooling is provided. Figures 4a and 4b illustrate an embodiment which is designed to provide heating through 360° using three separate peltier elements 38.

Figures 4a and 4b are side and end sectional views, respectively, of an alternative configuration of a heating/cooling assembly 15. In this example, three peltier elements 38 are arranged to face in different azimuthal directions in order to increase the range of angles through which heating/cooling can be provided. In this particular example the peltier elements are arranged so as to completely surround the heat sink 40 azimuthally in a triangular arrangement, as shown in Figure 4b. A continuous loop of contact element 18 surrounds the peltier elements 38 (this may be described as a single loop of contact element 18 and associated contact surface 22 or as a plurality of contact elements 18 and associated contact surfaces 22 joined together) and the heat sink 40 and provides an azimuthally continuous contact surface 22 (or plurality of contact surfaces 22). The heating/cooling assembly 15 is thus able to provide heating/cooling through 360°. The provision of 360° heating/cooling greatly increases the probability of achieving a good thermal contact between the contact surface 22 and tissue for a given position of the heating/cooling assembly 15 within the patient. In the example shown, three peltier elements 38 are provided, angled at 120° with respect to each other. However, in alternative configurations, fewer than three or more than three peltier elements 38 may be provided while maintaining the azimuthally continuous loop of contact element 18. For example, a similar effect to that achieved with the arrangement of Figures 4a and 4b could be achieved by replacing two of the three peltier elements 38 with insulating members that act simply to isolate the heat sink 40 from the surrounding contact element 18. In such a configuration, the heating or cooling would be provided solely by the remaining peltier element 38, but the high thermal conductivity of the contact element 18 would ensure that the temperature profile around the contact surface 22 would remain relatively uniform. However, the provision of multiple peltier elements 38 will generally increase the range and/or rate of heating/cooling that is possible relative to the case where only a single peltier element 38 is provided, and will also help to improve the uniformity of the temperature profile around the contact surface 22 further than may be possible using a single peltier element.

Temperature sensors 46/48 may be provided for measuring the temperature of the contact element 18 (and therefore of the contact surface 22) and/or of the heat sink 40. Measurements of the temperature of the contact element 18 can be used both to confirm proper operation of the device 10 and to improve the reliability with which measured or indicated patient response can be linked to heating or cooling provided by the heating/cooling assembly 15. Temperature measurements of the heat sink 40 can be used to confirm correct operation of the heating/cooling assembly 15. In addition, where the heat sink 40 is linked to a heat exchanger (see Figure 5), the measured temperature of the heat sink 40 can be used to control operation of the heat exchanger (in the case where an active heat exchanger is provided).

Figure 5 is a schematic side sectional view of an alternative configuration in which two heating/cooling assemblies 15 are provided in series. In the example shown, the heating/cooling assemblies 15 are of the type illustrated in Figures 3a and 3b, having a contact surface that spans 180°. In the example shown, the left hand heating/cooling assembly 15 has the contact surface semicircle oriented upwards and the right hand heating/cooling assembly 15 has the contact surface semicircle oriented downwards. In combination, the two heating/cooling assemblies therefore present a contact surface in all azimuthal directions. The arrangement shown is also provided with a heat exchanger 44 for controlling the temperature of the heat sink 40. In particular, the heat exchanger 44 is configured to prevent excessive heating or cooling of the heat sink, preferably ensuring that the heat sink temperature remains within a range that allows the peltier element 38 to operate efficiently. The heat exchanger 44 may be active, responding to temperature measurements of the heat sink 40, or passive. In the example shown in Figure 5, the heat exchanger 44 is passive and simply provides a high thermal conductivity link between the heat sink 40 of the two heating/cooling assemblies 15 and between the heat sinks 40 and the proximal end of the device 10.

The provision of temperature sensors has been described in the context of the example illustrated in Figures 4a and 4b. However, temperature sensors could be used in any of the described embodiments. The provision of more than one heating/cooling assembly 15 has been described with reference to Figure 5 using heating/cooling assemblies of the type described with reference to Figures 3a and 3b. However, multiple heating/cooling assemblies could be based on other types of heating/cooling assemblies, for example of the type described with reference to Figures 4a and 4b, and/or combinations of different types of heating/cooling assemblies having a variety of different relative contact surface azimuthal orientations relative to each other.

It is to be understood that appropriate methods, such as wiring or wireless, will be provided for connecting all of the electronic components described (e.g. the contact sensors, peltier elements, temperature sensors, electrical sensors, pH sensors, etc.) to appropriate power and control apparatus located outside of the patient, for example via an internal lumen of the catheter body 12.

The dimensions of the various elements of the device 10, and the degree of flexibility of the catheter body 12, are configured preferably so that the device can be inserted orally into the oesophagus while being well tolerated by the patient. Further preferably, the dimensions of the device are such that the device can be inserted nasally into the oesophagus while being well tolerated by the patient.

## Claims

1. A device for sensitivity testing, comprising:
a flexible catheter body having a distal end and a proximal end;
a peltier element;
a contact element located at or near the distal end of the catheter body that can be heated or cooled by applying a voltage to the peltier element; and
a contact sensor for detecting contact between a contact surface of said contact element and tissue to be tested.

2. A device according to claim 1, wherein the device further comprises a heat sink, said peltier element being in thermal contact with the heat sink on a first side of the peltier element and in thermal contact with said contact element on a second side of the peltier element, wherein the device optionally further comprises:
a thermally insulating member configured to surround at least a portion of said heat sink and thereby prevent or minimize conduction of heat between said heat sink and the tissue to be tested, said thermally insulating member being optionally formed from a plastic and said contact member being optionally formed from a metallic material; and/or
a heat sink temperature sensor for measuring the temperature of the heat sink

3. A device according to claim 2, configured such that, when a voltage is applied to said peltier element, heat is transferred from said heat sink to said contact element, or from said contact element to said heat sink, through said peltier element, wherein, optionally, the device is configured such that when a voltage is applied to said peltier element in a first sense, heat is transferred from said heat sink to said contact element, and when a voltage is applied to said peltier element in a second sense, heat is transferred from said contact element to said heat sink, wherein the first sense is opposite in polarity to the second sense.

4. A device according to any one of the preceding claims, wherein said contact surface is exposed to the region outside of the device.

5. A device according to any one of the preceding claims, wherein said contact sensor is capable of detecting contact with tissue at different positions around the periphery of said contact surface of the contact element.

6. A device according to any one of the preceding claims, further comprising:
an electrical sensor for measuring a change in an electrical property of a portion of tissue adjacent to the electrical sensor, wherein, optionally:
the electrical sensor is configured to measure a change in one or more of the following electrical properties of the portion of tissue: impedance, inductance, capacitance; and/or
the device comprises a plurality of said electrical sensors longitudinally separated from each other and/or from said contact surface.

7. A device according to any one of the preceding claims, further comprising:
a contact surface temperature sensor for measuring the temperature of the contact surface.

8. A device according to any one of the preceding claims, further comprising a heat exchanger configured to supply or remove heat from said heat sink to at least partially compensate for cooling or heating of said heat sink by the peltier element.

9. A device according to any one of the preceding claims, further comprising:
one or more further peltier elements each in thermal contact with the same contact element or a different contact element, wherein optionally:
a plurality of the peltier elements provided are located at different longitudinal positions along the catheter body; and/or
the device comprises a plurality of contact sensors each configured to detect contact between tissue and a different one of a plurality of contact surfaces associated with the plurality of different contact elements.

10. A device according to claim 9, wherein a plurality of the peltier elements are in thermal contact with contact elements having exposed contact surfaces that face in different, non-parallel directions, wherein optionally the plurality of exposed contact surfaces together form an azimuthally continuous 360 degree ring around the catheter body.

11. A device according to any one of the preceding claims, wherein the contact surface forms an azimuthally continuous 360 degree ring around the catheter body.

12. A device according to any one of the preceding claims, further comprising:
a pH sensor located at or near the distal end of the catheter body.

13. A device according to any one of the preceding claims, wherein:
the catheter body is sufficiently flexible to allow insertion of the device orally into the oesophagus while being well tolerated by the patient; and/or
the dimensions of the device are such that the device can be inserted nasally into the oesophagus while being well tolerated by the patient.

14. A device according to any one of the preceding claims, further comprising:
a remote control system comprising user operable means for interrupting a current applied to said peltier element while said device is in position within the patient, wherein:
the device optionally further comprises a remote control system comprising patient operable means for allowing a patient to provide an indication of a degree of sensation associated with heating or cooling provided by said peltier element while said device is in position within the patient.

15. A method of performing sensitivity testing, comprising:
inserting a flexible catheter body orally or nasally into a patient so that a contact element located at or near a distal end of the catheter body and in thermal contact with a peltier element is brought to a region to be tested within the oesophagus, the peltier element being capable of heating or cooling the contact element;
sensing whether a contact surface of the contact element is in contact with tissue at the region to be tested;
applying a voltage to the peltier element in order to apply heating or cooling to the tissue at the region to be tested if it is detected in said sensing step that the contact surface of the contact element is in contact with tissue at the region to be tested.
